# EUROPEAN PATENT APPLICATION

(11) **EP 4 728 981 A1**
(43) Date of publication of application: **22.04.2026**
(21) Application number: 24306741.0
(22) Date of filing: 18.10.2024
(51) Int. Cl.: A61B 5/08, F04B 45/06, G01N 33/00

(54) **A BREATH SIMULATOR FOR TESTING A BREATH ANALYZER**

(71) Applicant: BOYDSense, 31500 Toulouse (FR)
(72) Inventor: PELLETIER, Sébastien, Toulouse (FR); BOYÉ, Julien, Toulouse (FR)
(74) Representative: Grünecker Patent- und Rechtsanwälte PartG mbB

(57) **Abstract**

The present invention relates to a breath simulator (10) for testing a breath analyzer (100) configured to determine the presence and/or the concentration of at least one volatile organic compound (VOC). The breath simulator (10) comprises a pressure chamber (20) having an inlet port (24) configured to be connected to a fluid supply (200) and an outlet port (26) configured to be connected to the breath analyzer to be tested (100). The breath simulator (10) further comprises an expandable chamber (28) housed in the pressure chamber (20) and in fluid communication respectively with the inlet port (24) and the outlet port (26). The expandable chamber (28) is configured to be inflated by an injection of a predefined fluid volume inside the expandable chamber (28). The expandable chamber (28) is configured to eject the predefined fluid volume inside (5) the breath analyzer to be tested (100) when an overpressure, greater than a pressure in the expandable chamber (28), is generated inside the pressure chamber (20).

## Description

### Field of invention

The invention relates to a breath simulator for testing a breath analyzer, in particular a breath analyzer configured to determine the presence and/or the concentration of at least one volatile organic compound (VOC).

### Background of the invention

A breath simulator is an apparatus that mimic the inhalation and/or exhalation of a patient for the purpose of testing devices, like breath analyzers. Breath simulators enable automated testing and calibration of breath analyzers, without the need for human or animal subjects to generate a test breath.

Various types of breath simulators are known from the state of the art.

For example, ventilators or respirators are breath simulators based on mechanical ventilation methods. Mechanical ventilators or respirators may act like a pump, injecting and exhausting a volume of gas. Such mechanical ventilators or respirators generally comprise a compressible bellow and generate a flow of gas by means of a succession of positive and negative pressures. If the compressible bellow does not contain a sufficient volume of gas, the compressible bellow draws in atmospheric air. This may cause dilution of the gas, which may be unwanted in applications where a precise known concentration of the exhausted volume of gas is needed, for example for the calibration of breath analyzers. Moreover, mechanical ventilators or respirators often require complex mechanisms.

Another well-known type of breath simulators is the one used for the analysis of cigarette smoke. This type of breath simulator simulates the inhalation of cigarette smoke by a smoker, and then simulates the release of this smoke toward a smoke analyzer. To operate, this type of breath simulator requires a source of negative pressure, usually a vacuum pump, since the gas collection bag is filled by aspiration. To obtain the volume of gas corresponding to a puff from a cigarette smoker, this type of breath simulator dilutes the cigarette smoke with atmospheric air from another inlet, which is also connected to the gas collection bag.

The above-mentioned known breath simulators may thus operate by adding air from the atmosphere, thereby diluting the test breath. As a consequence, the concentration of the test breath is not known accurately. Hence, the known breath simulators may not accurately replicate the precise flow rates and gas compositions of human exhalation, particularly concerning specific concentrations of volatile organic compounds (VOCs).

Yet, in some applications, it is needed to know the concentration of the test breath, in particular for reproducing exhaled breath with a specific concentration or quantity of VOCs.

This is particularly the case, for instance, for testing breath diagnostic and monitoring analyzer, for disease such as cancer, chronic obstructive pulmonary disease (COPD), diabetes, sleep apnea, sepsis, metabolic dysfunction-associated steatohepatitis (MASH, also known as MAFLD, for metabolic associated fatty liver disease), asthma, cardiovascular diseases or COVID.

Thus, there is a need for improving the known breath simulators, in particular for providing a breath simulator capable to generate a known and reproducible breath test, in order to control better the quantity of VOCs exhaled in the breath analyzer.

Further, it would be desirable to simplify the construction and the maintenance of the breath simulator.

### Description of the invention

The object of the invention is achieved by means a breath simulator according to the independent claim 1. The breath simulator, for testing a breath analyzer configured to determine the presence and/or the concentration of at least one volatile organic compound (VOC), comprises a pressure chamber. The pressure chamber may be a housing isolated from the outside in which it is possible to modify the pressure, so that the pressure therein may be greater or smaller than the atmospheric pressure. The pressure chamber comprises an inlet port and an outlet port. In particular, the pressure chamber may be sealed such that fluid communication with the outside of the pressure chamber is only possible through the inlet port and the outlet port of the pressure chamber. The inlet port is configured to be connected to a fluid supply. The outlet port is configured to be connected to the breath analyzer to be tested. The pressure chamber is configured to resist to an increase of its internal pressure. In particular, the pressure chamber may be configured to resist to an increase of its internal pressure up to 50 hPa to 200 hPa (i.e. 50 mbar to 200 mbar), more in particular up to 150 hPa to 200 hPa (i.e. 150 mbar to 200 mbar), even more in particular up to 200 hPa (i.e. up to 200 mbar). The breath simulator further comprises an expandable chamber. In particular, the expandable chamber may be a fluid bag. The expandable chamber is housed in the pressure chamber. The expandable chamber is in fluid communication respectively with the inlet port and the outlet port. The expandable chamber is configured to be inflated by an injection of a predefined fluid volume inside the expandable chamber. The expandable chamber is also configured to eject the predefined fluid volume inside the breath analyzer to be tested when an overpressure, greater than a pressure in the expandable chamber, is generated inside the pressure chamber. The predefined fluid volume ejectable in a breath analyzer may be a test breath for testing the breath analyzer. In particular, the fluid is air.

The breath simulator is thus configured such that the predefined fluid volume is provided without requiring dilution of the fluid volume inside the expandable chamber. The concentration of the predefined fluid volume inside the expandable chamber is thus known. Hence, the predefined fluid volume to be ejected inside the breath analyzer is advantageously known as well. As a result, the breath simulator is capable of generating repeatedly a test breath having a known volume and preferably a known concentration of VOC. The breath simulator thus allows to automate user testing of a breath analyzer without the need for human breath and with a precise a reproducible test breath.

Moreover, the construction of the breath simulator only requires a limited number of components to operate and may be cost efficient to manufacture and easy to maintain.

In particular, the expendable chamber may be a flexible container. The expendable chamber may be able to contract, in particular only by an increase of pressure in the pressure chamber. The expendable chamber may be able to expand. The expendable chamber may expand to accommodate the fluid or gas and contract or compress to expel it, in particular for replicating human lung-like behavior. The pressure exerted by the respiratory muscles in a human body is stimulated in the breath simulator by the pressure inside the pressure chamber acting on the expendable chamber. In particular, the expendable chamber allows the breath simulator to be configured to mimic human exhalation. The expendable chamber may be adapted to hold fluids or gases. The expendable chamber may be adapted to transfer fluids or gases, in particular from the breath simulator to a breath analyzer. The expendable chamber may be pliable. The expendable chamber may be free of any rigid walls. The expendable chamber may comprise or be made of Tedlar^{®}, Kynar^{®}, Napophan^{®}, FlexFoil^{®}, or plastic, such as polypropylene, PTFE (Polytetrafluoroethylene), or silicone. The expendable chamber may comprise stainless steel. In particular, the expendable chamber is a fluid bag. The fluid bag may rely on pressure changes in the pressure chamber for contraction. The fluid bag may allow reproducing the gradual pressure release characteristic of human exhalation. It may provide a more realistic simulation of lung behavior than a bellows or rigid piston design, for instance. Using a fluid bag as the expandable chamber may thus better replicate the natural dynamics of a lung, enhancing the accuracy of breath simulation.

The outlet port of the breath simulator may be configured for connection to multiple breath analyzers for testing. This may allow simultaneous testing in parallel. This configuration may enable efficient and concurrent testing of multiple breath analyzers.

The breath simulator can be further improved according to various advantageous embodiments. The various embodiments of the invention may not be understood as being limitative. Any one or more of the features an embodiment the present invention may be combined with any one or more features of another embodiment of the present invention.

In one embodiment of the invention, the breath simulator may further comprise a compressor configured to be connected to a gas reservoir and configured to inject a volume of compressed gas into the pressure chamber via a compressor port of the pressure chamber. The compressor port may be distinct from the inlet port and the outlet port. Injecting a volume of compressed gas into the pressure chamber allows increasing the pressure in the pressure chamber.

In one embodiment of the invention, the breath simulator may further comprise a vent. The pressure chamber may be connected to the vent. The vent may be configured to exhaust air from the pressure chamber. The vent may allow decreasing the pressure in the pressure chamber.

In one embodiment of the invention, the expandable chamber may be configured to be inflated until an inflated state. In the inflated state of the expandable chamber, the expandable chamber may comprise a fluid volume representative of a human exhalation. The volume of the expandable chamber may be comprised between 0.05 liter and 8 liters. The volume of the expandable chamber may comprise a fluid volume representative of a human exhalation. However, the volume of the expandable chamber may be selected not to replicate the total pulmonary capacity, which is around 6 liters, but to provide an adequate sample fluid volume to reliably test the breath analyzer. In particular, the volume may focus on the portion of exhaled breath where volatile organic compounds (VOCs) are most concentrated. Specifically, the volume of the expandable chamber may allow for the analysis of end-tidal breath, typically around 0.5 liter, where the relevant biomarkers are typically concentrated.

In one embodiment of the invention, the expandable chamber of the breath simulator may be in fluid communication with the inlet port via a valve. The valve may be configured to eject the predefined fluid volume to the expandable chamber, in particular only the predefined fluid volume. It allows using the necessary and predefined fluid volume only. It allows avoiding gas wastes.

The valve may be configured to be in fluidic connection with a fluid supply providing a continuous flow of fluid.

The valve may be configured to selectively flow a fluid, in particular a fluid from the fluid supply, in the expandable chamber or outside the pressure chamber.

In one embodiment of the invention, the breath simulator may comprise a flow controller, in particular a mass flow controller, positioned upstream of the valve. The flow controller has the advantage of improving accuracy of the air flow. The flow controller allows repeatability for a reliable process. By accurately setting the air flow, the flow controller helps reducing energy consumption. The flow controller may comprise a flow meter. The valve may be a control valve and may be combined with the flow controller. It allows improving the control and the precision of the VOC concentration in the fluid.

In one embodiment of the invention, the valve may be a 3-way valve comprising one inlet port and two outlet ports. The inlet port of the 3-way valve may be configured to be in fluidic connection with a fluid supply. One of the two outlet ports of the 3-way valve may be in fluidic connection with the expandable chamber. The other of the two outlet ports of the 3-way valve may be configured to exhaust gas upstream of the expandable chamber. The 3-way valve valves allows providing versatile and precise flow direction and control. The 3-way valve allows injecting more precisely the volume of fluid into the expandable chamber.

In one embodiment of the invention, the breath simulator may further comprise a controller configured to control the valve according to the volume of fluid inside the expandable chamber. It allows controlling more precisely the breath test. This improves efficiency and may lower operational costs. The controller allows remote control and monitoring of the valve. The valve may be a control valve, in particular an electronic control valve. The control valve may be used to regulate the fluid flow by changing its valve parameters (e.g. size) as instructed by a signal from the controller.

In one embodiment of the invention, the breath simulator may further comprise a detecting means configured to detect an inflated state of the expandable chamber. The expandable chamber may contain a fluid volume corresponding to the predefined fluid volume in said inflated state. It allows ensuring that the inflated state is reached, in other words, that the predefined fluid volume has been correctly injected into the expandable chamber. It may further allow to control automatically the injection and/or ejection of the fluid into the expandable chamber. In the inflated state of the expandable chamber, the expandable chamber may comprise a fluid volume representative of a human exhalation. The detecting means may comprise a pressure sensor arranged upstream of the valve. The detecting means may comprise a proximity sensor, in particular arranged upstream of the valve. The detecting means may be coupled with a switch, in particular a mechanical lever switch, configured to commute the valve between a closed state of the valve and an open state of the valve. Alternatively or in combination, the detecting means may be an optical sensor. The optical sensor may be an optical pressure sensor. The optical sensor may be placed outside the expandable chamber, in particular outside the expandable chamber and the pressure chamber. The optical sensor may detect through a transparent wall of the expandable chamber, or a respective transparent wall of the expandable chamber and the pressure chamber.

In one embodiment of the invention, the expandable chamber may be configured to be in fluid communication with the breath analyzer to be tested via a proportional valve. The proportional valve may be arranged between the expandable chamber and the outlet port of the pressure chamber. An output flow exhausted by the breath simulator into a breath analyzer can be regulated with the proportional valve to simulate a profile of exhalation. It allows reproducing more accurately a breath a patient, in particular for the purpose of testing a breath analyzer. The breath simulator may comprise a flow controller, in particular a mass flow controller, positioned upstream of the proportional valve. The flow controller may be configured to regulate the flow of fluid upstream of the proportional valve. This may provide precise and stable control of the airflow, allowing for accurate simulation of exhalation in the breath analyzer, in particular for providing breath sample of few millimeters.

In one embodiment of the invention, the expandable chamber may be provided with only one aperture. The only one aperture may be in fluid connection with a valve connector. The valve connector may be arranged between the valve and the proportional valve. By minimizing the number of apertures, the hermeticity of the expandable chamber may be improved. It may allow reducing the risk of leakage and ensuring more accurate pressure control within the expandable chamber. Ultimately, it may enhance the reliability and precision of breath simulation.

In one embodiment of the invention, the breath simulator may comprise two or more valves or a controller configured to control the two or more valves of the breath simulator respectively and independently from one another. It allows controlling even more accurately the breath simulator and defining better the parameters of a breath test.

In one embodiment of the invention, the breath simulator may further comprise a heater, in particular a heater arranged to heat a fluid inside the pressure chamber. It advantageously allows setting the temperature of the predefined volume of the test breath to be injected in the breath analyzer. It may allow reproducing more accurately a specific breath parameter, namely the breath temperature. In particular, it may allow reproducing the temperature conditions similar to those of the human body. In one embodiment of the invention, the heater may be configured to heat the pressure chamber at a temperature comprised between 20 Celsius degree and 50 Celsius degree, in particular between 25 Celsius degree and 45 Celsius degree, more in particular between 32 Celsius degree and 42 Celsius degree, even more in particular between37 Celsius degree and 41 Celsius degree. The heater may allow that the temperature of the test breath in the breath simulator remains stable, in particular sufficiently warm to prevent the formation of cold spots that could potentially lead to condensation of water droplets. It may be desirable to avoid condensation for preventing altering the volatile organic compound (VOC) concentration in the test breath. In particular, by maintaining the pressure chamber at 40 Celsius degree, it may allow that the test breath retains its humidity profile, closely mimicking human breath conditions. It may result in consistent and reproducible test breath.

In one embodiment of the invention, the breath simulator may further comprise a fan inside the pressure chamber, in particular a fan arranged to move a fluid inside the pressure chamber. It may allow homogenizing the temperature inside the pressure chamber.

The object of the present invention is further achieved by means of a system comprising the breath simulator according to one of the preceding embodiments and a fluid supply, the fluid supply being in fluidic connection with the expandable chamber, and wherein the fluid supply is configured to inject a predefined volume of a breath sample inside the expandable chamber. The system allows injecting a predefined volume of a breath sample from the fluid supply inside the expandable chamber. The system is configured for testing and/or calibrating a breath analyzer in a reliable manner because the breath sample can be precisely and reproducibly generated by the system.

In one embodiment of the invention, the system is configured such that the breath sample comprises at least one volatile organic compound (VOC) with a known concentration. The system allows injecting a predefined volume of a breath sample from the fluid supply and containing specific VOCs in known concentration inside the expandable chamber. A breath sample comprising at least one VOC with a known concentration is particularly adapted for testing a breath analyzer. In particular, breath analyzers configured for measuring glycaemia of type 2 diabetics may advantageously be tested and/or calibrated by means of this system.

The fluid supply may comprise at least one fluid source provided with a respective regulator, the regulator being arranged between the fluid source and the expandable chamber. The fluid supply may comprise a gas mixer and at least two gas sources of different composition.

In one embodiment of the invention, the system may comprise at least two breath simulators and at least one fluid supply. The respective inlet ports of the at least two breath simulators may be in fluidic connection with the at least one fluid supply. The respective outlet ports of the at least two breath simulators may be configured to be connected to a respective breath analyzer. Such system may allow providing simultaneously at least two breath samples, and may thus be adapted for testing simultaneously at least two breath analyzers, in particular by means of one common fluid supply.

The object of the present invention is also achieved by a method for producing a test breath by means of a breath simulator, in particular according to one of the above embodiments of the breath simulator. The method comprises the steps of: connecting the breath simulator to a breath analyzer and a fluid supply, injecting a predefined fluid volume into an expandable chamber housed in a pressure chamber of the breath simulator, and ejecting the predefined fluid volume from the expandable chamber into the breath analyzer by generating a positive pressure in the pressure chamber. The method allows providing a predefined fluid volume without requiring dilution of the fluid volume inside the expandable chamber. The concentration of the predefined fluid volume inside the expandable chamber is thus known. Hence, the predefined fluid volume to be ejected inside the breath analyzer is advantageously known as well. As a result, the method allows generating repeatedly a test breath having a known volume and preferably a known concentration of VOC. The method may therefore automate user testing of a breath analyzer without the need for human breath and with a precise a reproducible test breath.

According to one embodiment, the method may comprise a step of generating a positive pressure in the pressure chamber, and ejecting the predefined fluid volume from the expandable chamber into the breath analyzer when the pressure in the pressure chamber has reached a predetermined threshold.

According to one embodiment, the method may comprise a step of comprising heating the predefined fluid volume with a predefined concentration at a temperature comprised between 30 Celsius degree and 45 Celsius degree, in particular between 37 Celsius degree and 41 Celsius degree. In particular, it may allow reproducing the temperature conditions similar to those of the human body.

According to one embodiment, the method may further comprise a cleaning step for cleaning the expandable chamber comprising injecting dry air in the expandable chamber. For instance, once the expandable chamber has been emptied, the expandable chamber can be filled with clean, dry air to remove any molecules remaining inside. It may allow preventing contamination, which could affect the VOC concentration, for instance when predefined fluid volumes of different concentrations are generated successively. It may allow extending the lifetime of the breath simulator.

The method may further comprise a cleaning step for cleaning the breath analyzer, said cleaning step comprising injecting a cleaning fluid in the breath simulator to clean the breath analyzer, in particular by means of the cleaning fluid ejected from the expandable chamber of the breath simulator into the breath analyzer. For instance, clean air can be circulated from the breath simulator through the breath analyzer to clean it after passing the breath test through it. Hence, the breath simulator may be advantageously adapted to clean the breath analyzer.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form, individually or in different combinations, solutions according to the present invention. The following described embodiments thus can be considered either alone or in an arbitrary combination thereof. Further features and advantages will become apparent from the following more particular description of the various embodiments of the invention, as illustrated in the accompanying drawings, in which like references refer to like elements, and wherein:
**Figure 1** is a scheme of a breath simulator according to a first embodiment of the invention.
**Figure 2** illustrates schematically a method for producing a test breath by means of the breath simulator of Figure 1.
**Figure 3** illustrates schematically different operational steps carried out in the breath simulator of Figure 1 to one embodiment of the present invention.

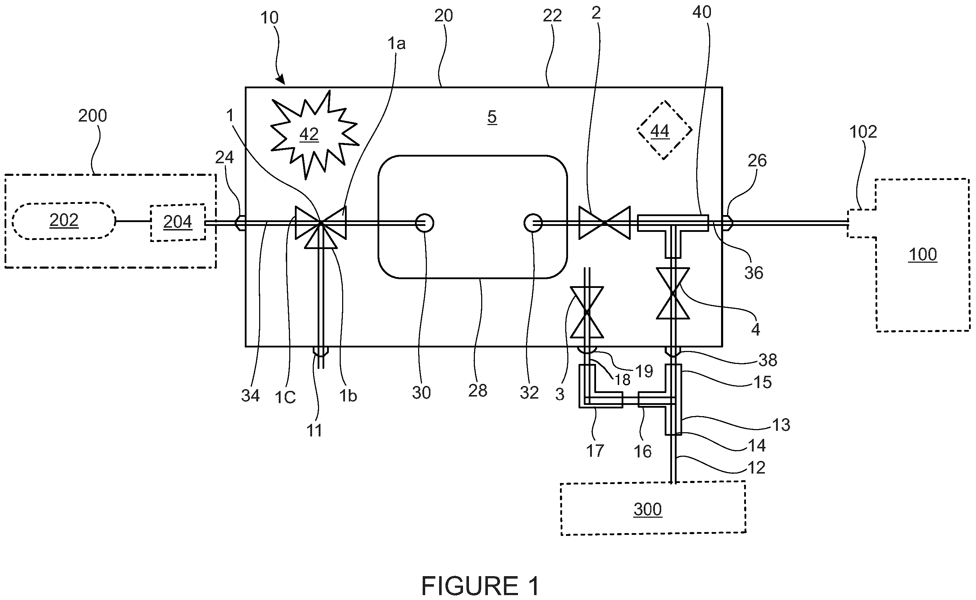

**Figure 1** shows a breath simulator 10. The breath simulator 10 is configured for testing a breath analyzer 100. The breath analyzer is configured to determine the presence and/or the concentration of at least one volatile organic compound (VOC).

The breath simulator 10 comprises a pressure chamber 20. The pressure chamber 20 is configured to resist to an increase of its internal pressure. The pressure chamber 20 may be formed by a hermetic case 22. The pressure chamber 20 comprises an inlet port 24 and an outlet port 26. Each of the inlet port 24 and the outlet port 26 may be respectively formed by hermetic feedthroughs in the hermetic case 22. The pressure chamber 20 is configured such that the pressure inside 5 the pressure chamber 20 may be set different with respect to an outside pressure. The pressure inside 5 the pressure chamber 20 may be greater or smaller than the atmospheric pressure.

The inlet port 24 is configured to be connected to a fluid supply 200. In the example of figure 1, the fluid supply 200 comprises a gas source 202, e.g. a gas tank, in fluid connection with a regulator 204. The regulator 204 is in fluid connection with the inlet port 24. In an alternative embodiment not represented, the fluid supply 200 may comprise a plurality of gas sources with respective regulators. The fluid supply 200 may further comprise a gas mixer downstream of the plurality of gas sources and regulators. The plurality of gas sources may respectively provide a gas with a different composition with one another. In one example, the fluid supply 200 may be configured to provide a continuous flow of fluid.

The outlet port 26 is configured to be connected to the breath analyzer 100 to be tested.

The breath simulator 10 comprises an expandable chamber 28. In the example of Figure 1, the expandable chamber 28 is a fluid bag 28. The expandable chamber 28 is housed in the pressure chamber 20. The volume of the expandable chamber 28 may be comprised between 0.05 liter and 8 liters, in particular between 0.05 liter and 2 liters. The expandable chamber 28 is in fluid communication respectively with the inlet port 24 and the outlet port 26. The expandable chamber 28 is provided with a first opening 30 and a second opening 32. In particular, the expandable chamber 28 may be provided with only two openings 30, 32. The first opening 30 of the expandable chamber 28 is in fluid communication with the inlet port 24 of the pressure chamber 20, in particular by means of a feedthrough 34. The second opening 32 of the expandable chamber 28 is in fluid communication with the outlet port 26 of the pressure chamber 20, in particular by means of a feedthrough 36. The respective feedthroughs 34, 36 may formed by a tube or a pipe, in particular by a pipe or a tube having an internal diameter inferior or equal to 10 millimeters, or inferior to 8 millimeters, or inferior to 6 millimeters, or inferior to 4 millimeters. The relatively small internal diameter may allow for precise control over the flow rate. The respective feedthroughs 34, 36 may be formed by a tube or a pipe having an internal diameter greater than 3 millimeters. The minimum internal diameter of 3 millimeters may ensure sufficient flow rate for the operation of the breath simulator 10.

In an alternative embodiment not represented, the expandable chamber 28 may be provided a single opening, instead of two openings 30, 32. The single opening may be connected to a connecting means configured to direct a fluid between the feedthrough 34, the feedthrough 36 and the single opening of the expandable chamber 28.

The expandable chamber 28 is in fluid communication with the inlet port 24 via a valve 1. The valve 1 is configured to eject a predefined fluid volume from the fluid supply 200 to the expandable chamber 28. In the example illustrated by Figure 1, the valve 1 is a three-way valve. The three-way valve 1 comprises two outlet ports 1a, 1b and one inlet port 1c. The first outlet port 1a of the three-way valve 1 is configured to eject fluid inside the expandable chamber 28 via the opening 30 of the expandable chamber 28. The second outlet port 1b of the three-way valve 1 is configured to exhaust fluid outside the pressure chamber 20, in particular via an outlet port 11 of the pressure chamber 20 distinct from the outlet port 26. The inlet port 1c of the three-way valve 1 is in fluidic connection with the fluid supply 200, in particular via the feedthrough 34.

The fluid simulator 10 may comprise a flow controller (not represented in Figure 1), in particular a mass flow controller, positioned upstream of the valve 1.

The fluid simulator 10 may comprise a controller (not represented in Figure 1) configured to control the valve 1 according to the volume of fluid inside the expandable chamber 28.

The expandable chamber 28 is configured to be in fluid communication via a valve 2 with the breath analyzer 100, in particular with a mouthpiece 102 of the breath analyzer. In particular, the valve 2 is a proportional valve. The valve 2 is arranged between the expandable chamber 28, in particular the opening 32 of the expandable chamber 28, and the outlet port 26 of the pressure chamber 20.

The expandable chamber 28 is configured to be inflated by an injection of a predefined fluid volume inside the expandable chamber 28. The expandable chamber 28 is configured to eject the predefined fluid volume inside the breath analyzer 100 to be tested when an overpressure, greater than a pressure in the expandable chamber 28, is generated inside 5 the pressure chamber 20.

To generate an overpressure inside 5 the pressure chamber 20, the pressure chamber 20 is connected to an air compressor 300. The air compressor is connected to the pressure chamber 20 via an opening 38, in particular a sealed opening 38, of the pressure chamber 20. The opening 38 of the pressure chamber 20 is respectively distinct form the inlet port 24 and the outlet port 26 of the pressure chamber 20. The opening 38 of the pressure chamber 20 is distinct from the outlet port 11. In the example of figure 1, the air compressor 300 is provided with a feedthrough 12. The feedthrough 12 may be a tube or a pipe, having in particular an internal diameter comprised between 5 millimeters and 12 millimeters, more in particular 7 millimeters and 9 millimeters. The feedthrough 12 is connected at one end to the air compressor 300 and at another opposite end to a T-shape tube 13. The T-shape tube 13 has three free ends 14, 15, 16. The T-shape tube 13 is connected at a first end 14 with the air compressor 300. The T-shape tube 13 is connected at a second end 15 with a valve 4. The T-shape tube 13 is connected at a third end 16 with a valve 3. The valve 3 is in fluid connection with the air compressor 300, in particular via a right angle tube 17. The shape of the right angle tube 17 is not limitative. Indeed, while the right angle tube 17 is a suitable example, other configurations may be employed to reduce flow resistance and minimize turbulence, thereby ensuring smoother airflow. For instance, the connection between the valve 3 and the air compressor 300 may alternatively be realized by means of a curved or gradually angled tube. The right angle tube 17 is connected to the third end 16 of the T-shape tube 13. In the example of figure 1, a feedthrough 18 connecting the valve 3 and the right angle tube 17 passes through an opening 19 of the pressure chamber 20. In the example of Figure 1, the opening 19 is distinct from the opening 38. In an alternative embodiment, not represented, the T-shape tube 13 and the right angle tube 17 may be housed inside 5 the pressure chamber 20 (rather than outside like in the illustrative example of figure 1). Hence, only one opening is necessary for passing the feedthrough 12 extended from the air compressor 300 towards the first end 14 of T-shape tube 13 located inside 5 of the pressure chamber 20. Such arrangement allows improving sealing of the pressure chamber 20 by limiting openings.

The valve 3 is arranged inside 5 the pressure chamber 20. The air compressor 300 is configured to be connected to a gas reservoir (not represented) and configured to inject a volume of compressed gas into the pressure chamber 20. By commuting the valve 3 in an open position, an overpressure can be generated inside 5 the pressure chamber 20 by means of the compressed gas supplied via the air compressor 300 inside 5 the pressure chamber 20.

In the example represented by Figure 1, the feedthrough 36 is provided with a T-tube 40. The T-tube 40 is arranged between the valve 2 and the outlet port 26. In the example represented by Figure 1, a valve 4 is provided between the T-tube 40 and the air compressor 300. The T-tube 40 is connected to the air compressor 300 via the opening 38 of the pressure chamber 20. Alternatively, the T-tube 40 may be connected to the air compressor an opening of the pressure chamber 20 distinct from the opening 38 of the pressure chamber 20.

The breath simulator 10 may further comprise a vent (not represented). The pressure chamber 20 may be connected to the vent. The vent may be configured to exhaust air from the pressure chamber 20. The vent may allow decreasing the pressure in the pressure chamber 20.

Optionally, the pressure chamber 20 may be provided with a heater 42. The heater 42 is arranged to heat the fluid inside 5 the pressure chamber 20. The heater 42 is configured, in particular controlled, to heat the pressure chamber 20 at a temperature comprised between 20 Celsius degree and 50 Celsius degree, in particular between 25 Celsius degree and 45 Celsius degree, more in particular between 32 Celsius degree and 42 Celsius degree, even more in particular between 37 Celsius degree and 41 Celsius degree and preferably at approximately 40 Celsius degree. The heater 42 allows that the temperature of the test breath in the breath simulator 10 remains stable, in particular sufficiently warm to prevent the formation of cold spots that could potentially lead to condensation of water droplets. It may be desirable to avoid condensation for preventing altering the volatile organic compound (VOC) concentration in the test breath. By maintaining the pressure chamber at approximately 40 Celsius degree, the heater 42 may ensure that the test breath retains its humidity profile, closely mimicking human breath conditions. This temperature control may allow the breath simulator to achieve consistent and reproducible test breath.

Optionally, the pressure chamber 20 may be provided with a fan 44. The fan 44 may be arranged to move the fluid inside 5 the pressure chamber 20. The fan 44 may be configured for homogenizing the temperature of the fluid contained inside 5 the pressure chamber 20.

The breath simulator 10 may comprise a controller (not represented). The controller is configured to control the different features, like the valves 1, 2, 3, 4, the heater 42 and/or the fan 44, of the breath simulator 10, in particular automatically, remotely and/or independently from one to another. The breath simulator 10 may be computer-controlled.

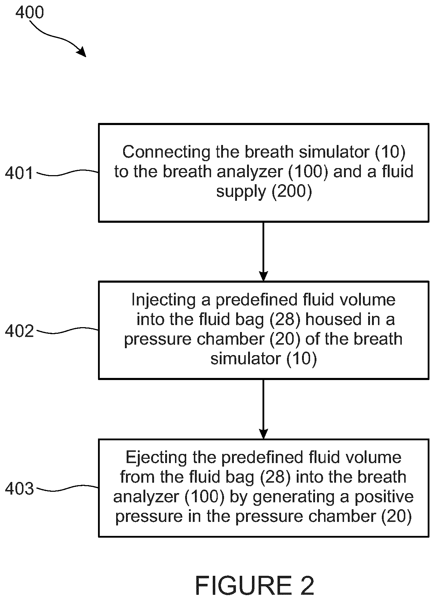

**Figure 2** illustrates schematically a method for producing a test breath by means of the breath simulator 10. In the following, elements with the same reference numeral already described and illustrated with respect to Figure 1 will not be described in detail again, and reference is made to Figure 1.

The method 400 for producing a test breath by means of the breath simulator 10 may comprise a first step 401 of connecting the breath simulator 10 to the breath analyzer 100 and a fluid supply 200.

The method 400 may comprise a second step 402 of injecting a predefined fluid volume into the expandable chamber 28 housed in the pressure chamber 20 of the breath simulator 10.

The method 400 may comprise a third step 403 of ejecting the predefined fluid volume from the expandable chamber 28 into the breath analyzer 100 by generating a positive pressure in the pressure chamber 20.

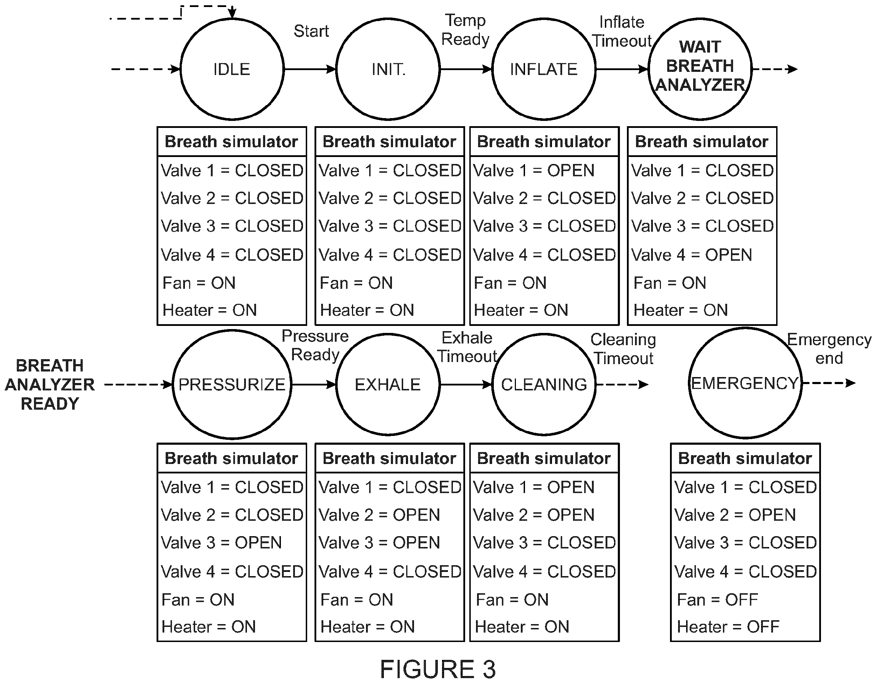

The method 400 for producing a test breath by means of the breath simulator 10 is further described in light of the different operational steps illustrated by Figure 3.

**Figure 3** illustrates schematically different operational steps carried out in the breath simulator 10 according to one embodiment of the present invention. In the following, elements with the same reference numeral already described and illustrated with respect to Figures 1 and 2 will not be described in detail again, and reference is made to Figures 1 and 2.

At an idle state, the valves 1, 2, 3, 4 of the breath simulator 10 are respectively in a closed position. The heater 42 and the fan 44 are activated ("ON") to heat at a predefined temperature the fluid inside 5 the pressure chamber 20.

In an initial state, following the idle state, the valves 1, 2, 3, 4 of the breath simulator 10 are still in a respectively in a closed position. The heater 42 and the fan 44 are still activated ("ON").

In an inflating state, following the initial state, the fluid inside 5 the pressure chamber 20 has reached the predefined temperature. The valve 1 of the breath simulator 10 is switched to an open position. The open position of the valve 1 allows injecting fluid from the fluid supply 200 into the expandable chamber 28, in particular via the inlet port 1c and the outlet port 1a of the three-way valve 1 shown by the example of Figure 1. At the same time, the valves 2, 3, 4 of the breath simulator 10 remains closed. The duration of step 1 is defined by the time needed for filing the expandable chamber 28 with the predefined fluid volume. The expandable chamber 28 is considered filled when the expandable chamber 28 contains the predefined fluid volume. When the expandable chamber 28 contains the predefined fluid volume, the expandable chamber 28 is in a so-called "inflated state". The inflated state of the expandable chamber 28 may be sensed by the detection of an overpressure on the feedthrough 34 and cause the valve 1 to switch to exhaust through the outlet port 1b. Alternatively of in combination, the inflated state of the expandable chamber 28 may be detected by a switch arranged in the pressure chamber 20. Alternatively of in combination, a contactor may be placed outside the pressure chamber 20 and measure through a transparent wall portion of the pressure chamber 20.

In a following state, the expandable chamber 28 has reached the inflated state. The valve 1 is switched to a closed position. The valves 2 and 3 remain closed. The valve 4 is switched to an open position.

When the breath analyzer 100 is ready, the pressure chamber 20 is pressurized. To do so, the valves 1 and 3 remain closed and the valve 4 is switched to a closed position while the valve 3 is switched to an open position. Compressed air is injected via the open valve 3 in the pressure chamber 200 to increase the pressure inside 5 of the pressure chamber 200. The pressure inside 5 of the pressure chamber 200 may be increased until a predefined inside pressure. The predefined inside pressure may be greater than the pressure of the air and atmosphere surrounding the breath simulator 1110. The predefined inside pressure shall be sufficient for applying pressure on the expandable chamber 28 so as to eject the predefined fluid volume contained inside the expandable chamber 28 via the valve 2.

When the predefined inside pressure is reached, a step of exhalation starts. In the step of exhalation, the valve 2 is switched to an open position. In particular, the valve 2 is a proportional valve. The opening of the proportional valve 2 may be proportionally controlled to reproduce a predefined test breath, for instance the typical flow profile of a human breath.

The valve 3 is maintained in the open position. The valves 1 and 4 are respectively kept closed. It allows ejecting the predefined fluid volume contained inside the expandable chamber 28 into the breath analyzer 100. The predefined fluid volume reproduces a test breath exhaled in the breath analyzer 100, e.g. via a mouthpiece 102 of the breath analyzer 100. The step of exhalation lasts a predetermined time, in particular until the predefined fluid volume is emptied from the expandable chamber 28.

Optionally, the method for operating the breath simulator 10 may comprise a cleaning step. In the cleaning step, the valves 1 and 2 are respectively in an open position. The valves 3 and 4 are respectively in a closed position.

From the initial state to the cleaning step, the heater 42 and the fan 44 may be activated to heat the fluid inside 5 the pressure chamber 20 to a predefined temperature.

Optionally, the method for operating the breath simulator 10 may comprise an emergency step. The emergency step may be manually activated by an operator. Alternatively or in combination, the emergency step may be automatically activated by a controller of the breath simulator 10. In the emergency step, the valves 1, 2 and 4 are respectively closed. The valve 3 is opened. The heater 42 and the fan 44 are respectively switched off.

## Claims

1. A breath simulator (10) for testing a breath analyzer (100) configured to determine the presence and/or the concentration of at least one volatile organic compound (VOC), the breath simulator (10) comprising:
a pressure chamber (20),
the pressure chamber (20) comprises an inlet port (24) and an outlet port (26),
the inlet port (24) is configured to be connected to a fluid supply (200) and
the outlet port (26) is configured to be connected to the breath analyzer to be tested (100),
the pressure chamber (20) is configured to resist to an increase of its internal pressure,
an expandable chamber (28),
the expandable chamber (28) is housed in the pressure chamber (20),
the expandable chamber (28) is in fluid communication respectively with the inlet port (24) and the outlet port (26),
the expandable chamber (28) is configured to be inflated by an injection of a predefined fluid volume inside the expandable chamber (28), and
the expandable chamber (28) is configured to eject the predefined fluid volume inside the breath analyzer to be tested (100) when an overpressure, greater than a pressure in the expandable chamber (28), is generated inside (5) the pressure chamber (20).

2. The breath simulator according to claim 1, wherein the expandable chamber (28) is in fluid communication with the inlet port (24) via a valve (1), the valve (1) being configured to eject the predefined fluid volume to the expandable chamber (28).

3. The breath simulator according to claim 2, further comprising a flow controller, in particular a mass flow controller, positioned upstream of the valve (1).

4. The breath simulator according to claim 2 or 3, wherein the valve (1) is a 3-way valve comprising one inlet port (1c) and two outlet ports (1a, 1b),
the inlet port (1c) of the 3-way valve (1) being configured to be in fluidic connection with a fluid supply (200),
one (1a) of the two outlet ports (1a, 1b) of the 3-way valve (1) being in fluidic connection with the expandable chamber (28) and
the (1b) other of the two outlet (1a, 1b) ports of the 3-way valve (1) being configured to exhaust gas upstream of the expandable chamber (28).

5. The breath simulator according to any of claims 2 to 4, comprising a controller configured to control the valve (1) according to the volume of fluid inside the expandable chamber (28).

6. The breath simulator according to any of the preceding claims, further comprising a detecting means configured to detect an inflated state of the expandable chamber (28), the expandable chamber (28) containing a fluid volume corresponding to the predefined fluid volume in said inflated state.

7. The breath simulator according to any of the preceding claims, wherein the expandable chamber (28) is configured to be in fluid communication with the breath analyzer to be tested (100) via a proportional valve (2), the proportional valve (2) being arranged between the expandable chamber (28) and the outlet port (26) of the pressure chamber (20).

8. The breath simulator according to any of the preceding claims, further comprising two or more valves and a controller configured to control the two or more valves (1, 2, 3, 4) of the breath simulator (10) respectively and independently from one another.

9. The breath simulator according to any of the preceding claims, further comprising a heater (42), in particular a heater (42) arranged to heat a fluid inside (5) the pressure chamber (20).

10. The breath simulator according to claim 9, wherein the heater (42) is configured to heat the pressure chamber (20) at a temperature comprised between 20 Celsius degree and 50 Celsius degree, in particular between 25 Celsius degree and 45 Celsius degree, more in particular between 32 Celsius degree and 42 Celsius degree, even more in particular between 37 Celsius degree and 41 Celsius degree.

11. The breath simulator according to any of the preceding claims, further comprising a fan (44) inside the pressure chamber (20), in particular a fan (44) arranged to move a fluid inside (5) the pressure chamber (20).

12. A system comprising the breath simulator (10) according to one of claims 1 to 11 and a fluid supply (200), the fluid supply (200) being in fluidic connection with the expandable chamber (28), and wherein the fluid supply (200) is configured to inject a predefined volume of a breath sample inside the expandable chamber (28).

13. The system according to claim 12, configured such the breath sample comprises at least one volatile organic compound (VOC) with a known concentration.

14. A method for producing a test breath by means of a breath simulator, in particular according to the breath simulator according to one of claims 1 to 11, the method comprising the steps of:
connecting the breath simulator to a breath analyzer and a fluid supply,
injecting a predefined fluid volume into an expandable chamber housed in a pressure chamber of the breath simulator, and
ejecting the predefined fluid volume from the expandable chamber into the breath analyzer by generating a positive pressure in the pressure chamber.

15. The method of claim 14, further comprising cleaning step for cleaning the expandable chamber comprising injecting dry air in the expandable chamber.
